# EUROPEAN PATENT APPLICATION

(11) **EP 1 153 940 A1**
(43) Date of publication of application: **14.11.2001**
(21) Application number: 99970404.2
(22) Date of filing: 12.10.1999
(51) Int. Cl.: C08F 8/00, C08F 26/02, A61K 31/785, A61K 9/28, A61K 47/30

(54) **POLYMER COMBINING WITH PHOSPHORIC ACID AND PREPARATION CONTAINING THE SAME**

(30) Priority: 12.10.1998 JP 28903198
(71) Applicant: CHUGAI SEIYAKU KABUSHIKI KAISHA, Tokyo, 115-8543 (JP)
(72) Inventor: MATSUDA, Katsuya Chugai Seiyaku Kabushiki Kaisha, Tokyo 115-8543 (JP); KUBOTA, Ryuji Chugai Seiyaku Kabushiki Kaisha, Tokyo 171-8545 (JP); TAKADA, Noriyuki Chugai Seiyaku Kabushiki Kaisha, Gotenba-shi, Sizuoka 412-8513 (JP)
(74) Representative: VOSSIUS & PARTNER
(86) International application number: JP9905596
(87) International publication number: WO0022008

(57) **Abstract**

Disclosed are a phosphate-binding polymer having a true specific gravity of 1.18 - 1.24, tablets that solely consist of the particles of a phosphate-binding polymer having an average particle size of no more than 400 µm, with at least 90% being occupied by particles no larger than 500 µm, and having a true specific gravity of 1.18-1.24 and a water content of 1 - 14%, or tablets that contain both the particles and crystalline cellulose and/or low substituted hydroxypropyl cellulose, and a process for producing such tablets.

The phosphate-binding polymer can be formulated as tablets either alone or in combination with specified additives. Whichever the case, the tablets have satisfactory hardness, contain the active ingredient in high proportion, have high phosphate-binding capability and exhibit rapid disintegrability in an acidic to neutral region while having little sensitivity to the strength of agitation. The tablets are excellent pharmaceutical preparations that undergo reduced variations in bioavailability in spite of movements within the digestive tracts and pH changes.

## Description

### TECHNICAL FIELD

This invention relates to a phosphate-binding polymer and tablets containing it, as well as a process for producing the tablets.

### BACKGROUND ART

Phosphate-binding polymers are non-absorptive polymers having a phosphate adsorbing capability and they are useful as remedies for hyperphosphatemia induced by renal hypofunction such as chronic renal failure. As described in, for example, U.S. Patent No. 5496545 (Japanese Domestic Announcement Hei 9-504782), phosphate-binding polymers are publicly known as polycationic polymer compounds comprising primary and secondary amines which are prepared by crosslinking polyallylamine with a crosslinking agent such as epichlorohydrin.

According typically to the USP, supra, phosphate-binding polymer preparations as remedies for hyperphosphatemia can be formulated into tablets using various additives including crystalline cellulose. However, said USP presents no specific example of tablet formulations. Although the present inventors attempted to produce tablets by blending various additives with the phosphate-binding polymers obtained by the method described in the USP, no tablets could successfully be produced.

Moreover, known adsorbents for oral administration, as exemplified by a calcium polystyrene sulfonate preparation (Kalimate™ manufactured by Nikken Chemicals Co., Ltd.), a sodium polystyrene sulfonate preparation (Kayexalate™ manufactured by Torii & Co., Ltd.), an adsorptive charcoal preparation (Kremezin™ manufactured by Kureha Chemical Industry Co., Ltd), a cholestyramine preparation (Questran™ manufactured by Bristol-Myers Squibb Co.) and a precipitated calcium carbonate preparation (manufactured by Emisu Yakuhin K.K.), are all in dosage forms of bulk powders, powder preparation or capsules containing powders. Namely, there have been reported no examples of oral adsorbents in the form of tablets.

### DISCLOSURE OF THE INVENTION

The phosphate-binding polymer of the present invention is preferably represented by the formula 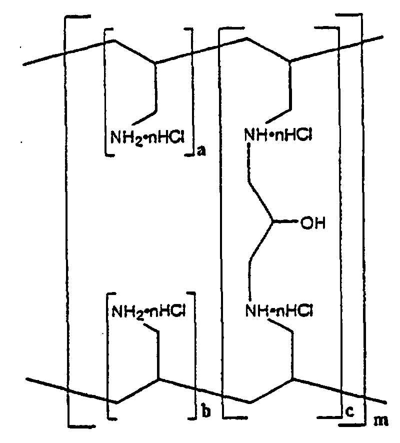 [where the molar ratio of (a+b) to c is from 45:1 to 2:1 and m is an integer] and has a true specific gravity of 1.18 - 1.24.

The tablets of the invention comprise the particles of a phosphate-binding polymer in optional presence of crystalline cellulose and/or low substituted hydroxypropyl cellulose; the particles have an average particle size of no more than 400 µm, with at least 90% being occupied by particles no larger than 500 µm, have a water content of 1 - 14% and are obtained by grinding a phosphate-binding polymer having a true specific gravity of 1.18 - 1.24, preferably 1.20 - 1.22. The tablets show adequate tablet hardness and exhibit rapid disintegrability and dispersibility, as well as high phosphate-binding capability.

The present invention also relates to a process for producing such tablets by compressing the above-defined particles of phosphate-binding polymer into tablets, optionally together with crystalline cellulose and/or low substituted hydroxypropyl cellulose.

When administered orally, phosphate-binding polymers adsorb phosphorus in foods and are excreted into the feces to thereby inhibit the absorption of phosphorus via the digestive tracts, thus controlling the serum phosphorus level. These phosphate-binding polymers are taken in a relatively large single dose, i.e., from 1 to 2 g. Because of reacting with water and thus swelling rapidly, the phosphate-binding polymers can be hardly taken as such. What is more, tablets prepared by compressing the conventional phosphate-binding polymers without additives have only insufficient tablet hardness, so it has been essential to incorporate substantial amounts of crystalline cellulose and/or low substituted hydroxypropyl cellulose.

Patients with dialysis who need be administered the phosphate-binding polymers as remedies for hyperphosphatemia are often required to take in limited amounts of water. It is therefore required to develop phosphate-binding polymer preparations in dosage forms that can be taken with small amounts of water. One of the promising dosage forms is tablets which can be reduced in size by compression and coated tablets are preferred since they will not disintegrate in the mouth and can be ingested smoothly. However, when processed into tablets by compressing, a phosphate-binding polymer alone gives only poor tablet hardness and thus cannot be processed as such into a tablet preparation. Further, due to the high hygroscopicity and swelling properties of the phosphate-binding polymer, it is also impossible to produce a phosphate-binding polymer preparation by a process comprising wet granulation using water or a binder solution containing alcohols, etc. and subsequent drying.

In order to overcome these problems, it has been required to develop a production process which comprises blending a powdery phosphate-binding polymer with powdery additives having excellent molding characteristics and compressing the obtained mixture. Such a preparation should be designed by taking into consideration changes in the disintegration properties and dispersibility accompanying the compression. Since a phosphate-binding polymer is to be taken in a relatively large single dose, special regard should be paid to give a preparation with a high content of the active component.

The present inventors attempted to produce phosphate-binding polymer preparations in the form of tablets by using the various additives described in USP 5496545, but they could not produce any favorable tablets which contain phosphate-binding polymer having a sufficient hardness, a rapid disintegrative dispersibility and an ability to bind to phosphate.

Under these circumstances, the present inventors have conducted intensive studies in order to solve the above-mentioned problems. As a result, they have successfully found that by using a phosphate-binding polymer having certain properties on its own, phosphate-binding polymer tablets substantially consisting of the phosphate-binding polymer in the absence of additives could be obtained that had adequate hardness and which exhibited rapid disintegrability and dispersibility, as well as high phosphate-binding capability in an acid to neutral region. The present invention has been accomplished on the basis of this finding.

### BRIEF DESCRIPTION OF THE DRAWINGS

Fig. 1 is a graph showing the disintegration characteristics of the phosphate-binding polymer preparation of Example 3 in terms of the relationship between the number of strokes by a disintegration tester and the tablet hardness); and
Fig. 2 is a graph showing the phosphate-binding profile of the phosphate-binding polymer preparation of Example 4.

### MODE FOR CARRYING OUT THE INVENTION

The present inventors found that excellent characteristics were exhibited by tablets that contained the particles of a phosphate-binding polymer either alone or in combination with crystalline cellulose and/or low substituted hydroxypropyl cellulose as specified additives, provided that the phosphate-binding polymer particles had a true specific gravity of 1.18 - 1.24, preferably 1.20-1.22, an average particle size of no more than 400 µm, preferably no more than 250 µm, with at least 90% being occupied by particles no larger than 500 µm, preferably no larger than 300 µm, and a water content of 1 - 14%. The stated values of true specific gravity were measured with a true specific gravity meter Accupyc Model 1330 of Shimadzu Corp.

The phosphate-binding polymer to be used in the invention may be prepared on the basis of the process described in USP 5496545, supra. In that process, a specified polymer is crosslinked with a specified crosslinker in the presence of solvent water. According to the present invention, the phosphate-binding polymer having a true specific gravity in the stated range can be produced by replacing water with a mixed solvent system consisting of water and acetonitrile in a volume ratio which usually ranges from 10:90 to 90:10, preferably from 40:60 to 60:40.

The thus obtained phosphate-binding polymer is dried and ground into particles having an average particle size of no more than 400 µm, preferably no more than 250 µm, with at least 90% being occupied by particles no larger than 500 µm, preferably no larger than 300 µm. The particles are then adjusted to 1 - 14% in water content. Among the phosphate-binding polymers thus prepared, a crosslinked polymer obtained by treating polyallylamine with epichlorohydrin is particularly suitable for use in the present invention. This polymer is represented by the formula 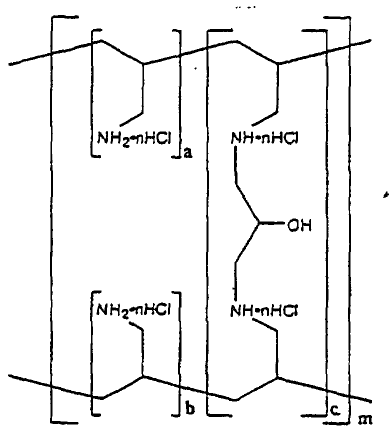 [where the molar ratio of (a+b) to c is from 45:1 to 2:1, preferably from 20:1 to 4:1, more preferably from about 10:1 to 8:1, the most preferably about 9:1, and m is an integer].

Since the phosphate-binding polymer of the invention is a crosslinked polymer, m in the above formula is a large integer that represents the network structure of the crosslinked and extended polymer and can theoretically be as great as 1 x 10⁷. Since this polymer is crosslinked in a network, each of the particles into which it has been ground is in effect a single molecule; therefore, the molecular weight of the polymer is equivalent to the weight of an individual polymer particle.

If the true specific gravity of the phosphate-binding polymer is greater than 1.24, tablets compressed from this polymer alone do not have adequate hardness. Phosphate-binding polymers whose true specific gravity is less than 1.18 are not suited to handling in industrial applications. Particles having a greater average particle size than 400 µm are not preferred since they do not have the necessary sufficient hardness to be compressed into tablets. If the water content of the phosphate-binding polymer is less than 1%, it again fails to have the necessary sufficient hardness to permit compression into tablets and if any tablets are formed, their surfaces are liable to abrasion. If the water content of the phosphate-binding polymer exceeds 14%, it has sufficient hardness to permit compressing into tablets but, on the other hand, the resulting tablets undergo plastic deformation to become no longer suitable as pharmaceutical preparations. In order that the tablets can be taken more smoothly, they must have a hardness that gives a reading of at least 6 KP on a tablet hardness meter and a surface strength that gives a weight loss of no more than 1% in a friability test (100 revolutions). What is more, the tablets should not undergo plastic deformation. To meet these requirements, the phosphate-binding polymer must have a water content in the range of 1 - 14%. The expression "a water content of 1-14%" as used herein means that after drying at 105°C for 16 hours, the polymer weight is reduced by 1 - 14%. The preferred drying weight loss of the phosphate-binding polymer ranges from 2 to 14%. If the phosphate-binding polymer per se absorbs moisture in the course of grinding to give a water content of 1 - 14%, there is no particular need to adjust the water content of the polymer and it may be directly used to prepare the tablets of the present invention.

The phosphate-binding polymer may be ground with any known model of mills such as an impact mill that can yield particles no larger than 500 µm and produce an average particle size of no more than 400 µm.

The water content of the phosphate-binding polymer can be adjusted either with a moisture-adjusting agent such as a saturated aqueous solution of sodium chloride (25°C, RH 75.3%), a saturated aqueous solution of calcium chloride (25°C, RH 84.3%) or a saturated aqueous solution of magnesium nitrate (25°C, RH 52.8%) or by letting the polymer absorb moisture in the air atmosphere. The phosphate-binding polymer of the desired water content can also be prepared by performing the drying step in its production process in such a way as to give a water content of 1 - 14%.

The crystalline cellulose to be used in the invention is not limited in any particular way and use may be made of one that gives a weight loss of no more than 7% after drying at 105°C for 3 hours. Preferably, commercial products such as Avicel™ PH101, PH102, PH301, PH302 and Ceolus™ KG-801 (all manufactured by Asahi Chemical Industry Co., Ltd.) may be used either alone or in admixture.

The low substituted hydroxypropyl cellulose that can be used in the invention is such that it has 5.0 - 16.0 wt% substitution by hydroxypropoxyl groups (-OC₃H₆OH).
Preferred examples of such low substituted hydroxypropyl cellulose are commercial products such as LH-11, LH-21 and LH-31 (manufactured by Shin-Etsu Chemical Co., Ltd.) that may be used either alone or in admixture.

The amounts of the crystalline cellulose and/or low substituted hydroxypropyl cellulose that are optionally added to the phosphate-binding polymer in the present invention may be set at any values in consideration of the dose of the phosphate-binding polymer to be taken as an oral preparation and the ease with which it can be taken. In a preferred embodiment, for example, the crystalline cellulose or the low substituted hydroxypropyl cellulose is used in an amount of at least 10 wt%, more preferably at least 30 wt%, based on the weight of the phosphate-binding polymer having an average particle size of no more than 250 µm, at least 90% being occupied by particles no larger than 300 µm, and a water content of 1 - 14%. If both of the crystalline cellulose and the low substituted hydroxypropyl cellulose are added, it is preferable that the sum of the contents of these components is 10 wt% or more, preferably 30 wt% or more. From the viewpoint of the administration properties, etc, of the preparation, the upper limit of the content of the crystalline cellulose and/or the low substituted hydroxypropyl cellulose is in the range of 50-200 wt%.

Since the phosphate-binding polymer, the crystalline cellulose and the low substituted hydroxypropyl cellulose produce great frictional forces, it is recommended that a hardened oil be used in a continuous tableting process in order to reduce the load on the tableting machine due to the jarring action of punches. A useful hardened oil is a commercial product such as Lubriwax™ manufactured by Freund Industrial Co., Ltd.

To produce the phosphate-binding polymer tablets of the present invention, the crystalline cellulose and/or low substituted hydroxypropyl cellulose, together with fillers (e.g. lactose, sucrose or mannitol), a lubricant (e.g. magnesium stearate or polyethylene glycol) and other additives conventionally employed in the art, perfumes, coloring agent etc. may be added and mixed with the phosphate-binding polymer and the resulting blend is compressed into tablets.

The phosphate-binding polymer tablets of the invention may be further processed into film-coated tablets by coating their surfaces with a film. In the film coating process, use may be made of water-soluble film bases such as hydroxypropyl methylcellulose and acrylic acid copolymers. Use of hydroxypropyl methylcellulose is particularly preferred.

The following preparations and examples are provided for further illustrating the present invention but are in no way to be taken as limiting.

### [Preparation 1]

Polyallylamine was subjected to crosslinking polymerization in a water/acetonitrile (ca. 50:50 w/w) mixed solvent with epichlorohydrin being added as a crosslinker, thereby producing a polycationic phosphate-binding polymer in which about 40% of the primary amine (81.2 mol%) and secondary amine (18.8 mol%) formed hydrochlorides. The polymer was then vacuum-dried to give a dry powder, which was ground with an impact mill into particles of water-containing phosphate-binding polymers (true specific gravity, 1.209 - 1.211; water content, 2.1-2.5%; under 300 µm size = 99.0 - 99.6%).

### [Preparation 2]

Polyallylamine was subjected to crosslinking polymerization in water with epichlorohydrin being added as a crosslinker, thereby producing a polycationic phosphate-binding polymer in which about 40% of the primary amine (81.2 mol%) and secondary amine (18.8 mol%) formed hydrochlorides. The polymer was then dried with air to give a dry powder, which was ground with an impact mill into particles of a water-containing phosphate-binding polymer (true specific gravity, 1.253; water content, 3.6-3.8%; under 300 µm size = 99.3 - 99.7%).

### [Example 1]

The particles of the water-containing phosphate-binding polymers obtained in Preparation 1 (to give true specific gravities of 1.209 - 1.211) and those of the polymer obtained in Preparation 2 (to give a true specific gravity of 1.253) were compressed under various static pressures of 500 - 1750 kg into tablets of 10 mm^{φ} each weighing 300 mg. These tablets were measured for hardness with a hardness meter (Pharmatest). The results are shown in Table 1.

**Table 1**

| | Preparation 1 | | | Preparation 2 | |
|---|---|---|---|---|---|
| True specific gravity | 1.209 | 1.211 | 1.211 | 1.253 | 1.253 |
| Water content | 2.5% | 2.1% | 2.1% | 3.6% | 3.8% |
| Under 300 µm size | 99.0% | 99.6% | 99.3% | 99.7% | 99.3% |
| Compressing pressure: 500 kg | 2.1 KP | 4.7 KP | 2.0 KP | 0.5 KP | 0.8 KP |
| 750 kg | 5.1 KP | 9.2 KP | 4.0 KP | 0.8 KP | 1.5 KP |
| 1000 kg | 10.8 KP | 11.6 KP | 8.5 KP | 1.3 KP | 2.5 KP |
| 1250 kg | 13.1 KP | 19.0 KP | 11.2 KP | 2.2 KP | 3.5 KP |
| 1500 kg | 19.5 KP | 20.0 KP | 13.8 KP | 2.6 KP | 4.6 KP |
| 1750 kg | 23.9 KP | 24.3 KP | 15.5 KP | 3.6 KP | 5.6 KP |

As one can see from Table 1, none of the tablets compressed solely from the phosphate-binding polymer with a true specific gravity of 1.253 had adequate hardness (≥ 6 KP) irrespective of the compressing pressure applied; on the other hand, the tablets compressed from the phosphate-binding polymers with true specific gravities of 1.209-1.211 had adequate hardness when they were compressed at pressures of 1000 kg and more.

### [Example 2]

Two hundred milligrams of the particles of the water-containing phosphate-binding polymer with a true specific gravity of 1.209 which was obtained in Preparation 1 were mixed with 100 mg of an additive crystalline cellulose (Avicel™ PH101 of Asahi Chemical Industry Co., Ltd.) and the blend was compressed under static pressures of 500 kg, 750 kg and 1000 kg into tablets of 10 mm^{φ} each weighing 300 mg.

These tablets were measured for hardness with a hardness meter. The tablets compressed at a pressure of 750 kg were also tested with a disintegration tester (Toyama sangyo) using water as a test fluid. The results of both tests are shown in Table 2.

**Table 2**

| | Tablet hardness | Disintegration time |
|---|---|---|
| Compressing pressure: 500 kg | 5.7 KP | |
| 750 kg | 9.0 KP | 20 seconds |
| 1000 kg | 13.6 KP | |

As one can see from Table 2, the tablets compressed from the blend of the phosphate-binding polymer and crystalline cellulose at pressures of 750 kg and more had hardness values of at least 6 KP and exhibited rapid disintegrability.

### [Example 3]

To 767.7 g of the particles of the water-containing phosphate-binding polymer with a true specific gravity of 1.209 that was obtained in Preparation 1, 349.5 g of crystalline cellulose, 5.6 g of a hardened oil (Lubriwax™ 101 of Freund) and 2.2 g of a lubricant magnesium stearate (Nitto Kasei) were added and the ingredients were mixed together. The resulting blend was set on a single-action tableting machine (Model N-30 of Okada Seiko) and compressed at 1750 kg into tablets of 10.5 mm^{φ} each weighing 375 mg. This yielded uncoated tablets each containing about 250 mg of the dry phosphate-binding polymer.

These tablets were measured for hardness with a hardness meter (Contester), which read a value of 10.9 KP. They showed a disintegration time of 67 seconds (test fluid, water).

The tablets each containing 250 mg of the phosphate-binding polymer were coated with a film comprising 8.25 mg of hydroxypropyl methylcellulose 2910 (HPMC TC-5-RW of Shin-Etsu Chemical Co., Ltd.), 1.26 mg of polyethylene glycol 6000 (Nippon Oil and Fats Co., Ltd.), 1.8 mg of titanium oxide (A-100 of Ishihara Sangyo Kaisha, Ltd.) and 0.69 mg of talc. The coating machine was Doria Coater Model DRC-500 of Powrec Corporation.

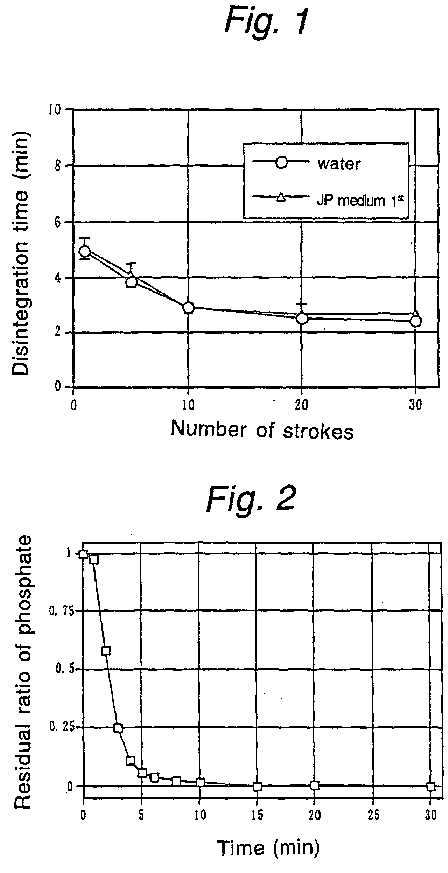

The film-coated tablets were tested with a disintegration tester at 1 - 30 strokes per minute using two test solutions (pH 1.2; medium 1^{st} specified in Japan Pharmacopoeia and water). The test results are shown in Fig. 1.

As one can see from Fig. 1, the phosphate-binding polymer preparation showed rapid disintegrability in an acidic to neutral region without being affected by the strength of agitation (number of strokes).

### [Example 4]

Four of the coated tablets prepared in Example 3 each containing 250 mg of the phosphate-binding polymer were subjected to the following simulated evaluation of drug efficacy: 4.7 g of sodium chloride, 21.3 g of N,N-bis(2-hydroxyethyl)-2-aminoethanesulfonic acid and 0.544 g of potassium dihydrogenphosphate were dissolved in water; after pH adjustment to 7, the solution was warmed to 37°C; 200 ml of the thus prepared test solution was stirred with the paddles rotating at 100 rpm to measure the phosphate-binding capability of the polymer. When the tablets disintegrated, the phosphate-binding polymer dispersed to adsorb the phosphate, causing the residual concentration of the phosphate in the test solution to vary with time. The initial phosphate level in the test solution was taken as 1 and the value at the completion of phosphate adsorption as 0. The results of the measurement are shown in Fig. 2.

Obviously, the phosphate-binding polymer preparations exhibited rapid phosphate binding capability.

### INDUSTRIAL APPLICABILITY

The phosphate-binding polymer of the present invention can be formulated as tablets irrespective of whether it is used alone or combined with additives. Whichever the case, the tablets have satisfactory hardness, contain the active ingredient in high proportion, have high phosphate-binding capability and exhibit rapid disintegrability in an acidic to neutral region while having little sensitivity to the strength of agitation. Therefore, the tablets of the invention are excellent pharmaceutical preparations that undergo reduced variations in bioavailability in spite of movements within the digestive tracts and pH changes.

## Claims

1. A phosphate-binding polymer that is represented by the formula 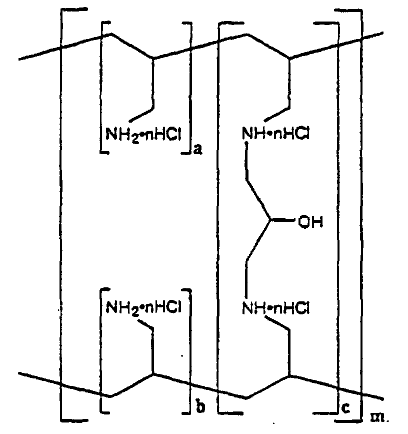 [where the molar ratio of (a+b) to c is from 45:1 to 2:1 and m is an integer] and which has a true specific gravity of 1.18 - 1.24.

2. The phosphate-binding polymer according to claim 1 which has a true specific gravity of 1.20 - 1.22.

3. The phosphate-binding polymer according to claim 1, wherein the molar ratio of (a+b):c is from 20:1 to 4:1.

4. A tablet comprising the particles of a phosphate-binding polymer having an average particle size of no more than 400 µm, with at least 90% being occupied by particles no larger than 500 µm, and having a true specific gravity of 1.18 - 1.24 and a water content of 1- 14%.

5. The tablet according to claim 4 which has a true specific gravity of 1.20- 1.22.

6. The tablet according to claim 4, 5 or 6, wherein said particles of a phosphate-binding polymer have an average particle size of no more than 250 µm, with at least 90% being occupied by particles no larger than 300 µm.

7. The tablet according to any one of claims 1 - 6 which further contains crystalline cellulose and/or low substituted hydroxypropyl cellulose.

8. The tablet according to claim 7, wherein the content of the crystalline cellulose and/or low substituted hydroxypropyl cellulose is at least 10 wt% of the weight of the phosphate-binding polymer.

9. The tablet according to claim 7 or 8, wherein the low substituted hydroxypropyl cellulose has 5.0 - 16.0 wt% substitution by hydroxypropoxyl groups.

10. The tablet according to any one of claims 4 - 9, wherein the phosphate-binding polymer is the one described in U.S. Patent No. 5496545.

11. The tablet according to any one of claims 3 - 9, wherein the phosphate-binding polymer is one that is obtained by allowing epichlorohydrin to act on polyallylamine in a water/aetonitrile mixed solvent system so that the polyallylamine is crosslinked.

12. The tablet according to any one of claims 4 - 11 which further contains a hardened oil.

13. The tablet according to any one of claims 4 - 12 which is coated on the surface with a water-soluble film base.

14. A process for producing phosphate-binding polymer tablets which comprises:
grinding a phosphate-binding polymer having a true specific gravity of 1.18 - 1.24 into particles having an average particle size of no more than 400 µm, with at least 90% being occupied by particles no larger than 500 µm, said phosphate-binding polymer being either polyalllamine or obtained by crosslinking the same;
adjusting the phosphate-binding polymer particles to a water content of 1 - 14%;
mixing the particles with crystalline cellulose and/or low substituted hydroxypropyl cellulose; and
compressing the mixture into tablets.

15. The process according to claim 14, wherein said phosphate-binding polymer is ground into particles having an average particle size of no more than 250 µm, with at least 90% being occupied by particles no larger than 300 µm.
